# EUROPEAN PATENT SPECIFICATION

(11) **EP 0 481 041 B1**
(45) Date of publication and mention of the grant of the patent: **11.01.1995**
(21) Application number: 91908104.2
(22) Date of filing: 08.04.1991
(51) Int. Cl.: C07C 69/704, C07C 67/08, C08K 5/11

(54) **CITRATE ESTER COMPOSITIONS AND PROCESSES FOR THEIR PREPARATION**
CITRATESTERZUSAMMENSETZUNGEN UND VERFAHREN ZU IHRER HERSTELLUNG
COMPOSITIONS D'ESTER DE CITRATE ET PROCEDES DE PREPARATION

(30) Priority: 02.05.1990 US 517863
(43) Date of publication of application: 22.04.1992
(73) Proprietor: OWENS-CORNING FIBERGLAS CORPORATION, Toledo, Ohio 43659 (US)
(72) Inventor: ROSS, Louis, Ralph, Newark, OH 43055 (US); KRUMLAUF, Paul, Richard, Thornville, OH 43076 (US); WILSON, Edward, Langer, Newark, OH 43055 (US)
(74) Representative: West, Alan Harry
(86) International application number: US9102350
(87) International publication number: WO9117136

(56) References cited:
- EP-A- 0 199 131
- US-A- 555 534
- US-A- 4 710 532

## Description

The present invention provides improved surface smoothness in unsaturated polyester resin compositions that contain low-profile additives. More specifically, these unsaturated resin compositions contain low-profile additives and compatible compounds. The present invention relates in particular to citrate ester compatible compounds and the processes for their preparation.

Unsaturated polyester resin compositions are finding increased use in the automotive industry as compositions from which component parts especially body panels can be molded. These compositions contain, in addition to the unsaturated polyesters, so-called "low-profile" additive components which are thermoplastic polymers that act to prevent undesirable shrinkage as the composition is being molded into a thermoset article. Low-profile additives are added to unsaturated polyester compositions for the purpose of obtaining a composition which can be molded into thermoset articles, where the surfaces of the molded articles truly reflect the surface characteristics of the mold.

Two types of low-profile systems are commonly used commercially, one- pack and two-pack. In one-pack systems, the polyester, styrene and low-profile additive components are mutually compatible, i.e., no gross separation occurs when a mixture is allowed to stand. In contrast, two-pack systems form distinct phases if the components are allowed to stand. These need to be mixed immediately prior to use. In either case phenomena occur that allow these resins to microscopically compensate for shrinkage.

It is the ability of the low-profile resins to compensate for shrinkage that leads to the usefulness of these resins. This shrinkage compensation is largely a result of a micro-phase separation that occurs in these unsaturated polyester resin systems. The micro-phase separation occurs during the cure phase for both the one-pack and two-pack systems.

Prior to cure the low-profile additive is at least partly soluble in the polyester/styrene solution. As the polyester/styrene mixture crosslinks, the low-profile thermoplastic additive and copolymer become increasingly less compatible and a two-phase (domain-matrix) type morphology results. This micro-phase separation leads to the formation of a porous structure as the opposing internal stresses of thermal expansion and polymerization shrinkage occur. In many unsaturated polyester resin compositions the porous structure is a result of microfracturing of the curing resins which gives rise to void formation. Unsaturated polyester resins have been developed which have essentially zero shrinkage and which, in fact, expand upon curing.

In addition to unsaturated polyester resins, the sheet molding compound formulations typically contain other ingredients including, for example, chemical thickeners. In such systems, an alkaline material such as magnesium oxide or magnesium hydroxide is added to, for example, an uncured polyester along with fillers, glass fiber, and other standard materials. The alkaline material interacts with the residual acidity in the polyester and, usually, the low-profile additive to build viscosity. This process is referred to as maturation and usually takes several days. If two-pack resin systems are used, care has to be taken to avoid gross phase separation. After the maturation process is complete, the thickened systems are handlable and can easily be placed into compression molds either by hand or by machine.

Although the use of low-profile additives as described as three component mixtures do effect some degree of improvement in the anti-shrinkage characteristics of the unsaturated polyester compositions, it has been found that significant improvements could yet be made on surface smoothness and processing characteristics.

Low-profile resins have been described that contain unsaturated polyester resins, thermoplastic low-profile additives, and a polymerizable monomer, usually styrene. In addition to these components other materials have been added to low-profile systems to improve specific properties.

U.S. Patent No. 4,622,354 describes "phase stabilizing agents" that comprise a select group of compounds from three classes: fatty acids, dimer acids and polyester polyols. When used in an SMC formulation where the thermoplastic low-profile additive is polymethylmethacrylate and a urethane prepolymer is included, the phase stabilizing agent reduces the gross separation that occurs during the maturation process. The resin compositions described by Iseler et al. are two-pack systems that formerly phase-separated during maturation prior to the addition of the phase stabilizers.

U.S. Patent No. 4,473,544 describes an anti-shrink additive with a tri- or tetrafunctional polyether condensation product of propylene oxide on a triol or tetrol wherein the condensation product is acidified in such a manner that it possesses at least one terminal acidic functional group per elementary molecule. This material is used as a low-profile additive.

U.S. Patent No. 4,555,534 describes low-shrink pigmentable unsaturated polyester resins which comprises a polyester resin comprising the reaction product of an olefinically unsaturated dicarboxylic acid or anhydride and a polyol, an olefinically unsaturated monomer, a thickening agent, a pigment, a carboxylated vinyl acetate polymer low-profile additive, and a surface active compound. The above patent describes low-shrink resins having improved uniformity of pigmentation in internally pigmented thickened polyester molding compositions. These pigmentable resin systems are low-shrink, and not low-profile. The surface quality of these pigmentable systems is considerably inferior to surfaces required for automotive appearance applications.

Although the use of low-profile additives and thickening agents, as described, do effect some degree of improvement in the antishrinkage and surface smoothness characteristics of the unsaturated polyester compositions, they are unable to achieve the degree of surface smoothness required of today's thermoset molded articles.

Reference should be made also to EP-A-0 199 131 which describes surfactants derived from citric acid and which find utility in toiletries and foods and in the textile industry. Such surfactants have the general formula 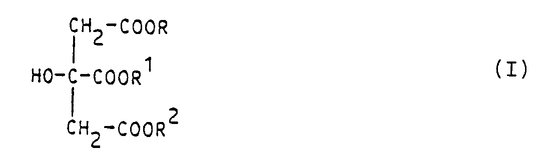
wherein:
R, R¹, R² are equal or different from each other and are H, an alkaline metal, alkaline-earth metal or -NH4 cation, a cation of an organic ammonium base or a group having the formula:

-Aₙ - R³ (II)

wherein:
A is a C₂-C₄ oxyalkylene group, n is a number ranging from 1 to 20 and R³ is a C₈-C₂₀ alkyl group, at least one of said R, R¹, R² being an -A ₙ-R³ group, and wherein the OH group can be esterified or etherified.

In one aspect, the present invention provides an ester of the general formula 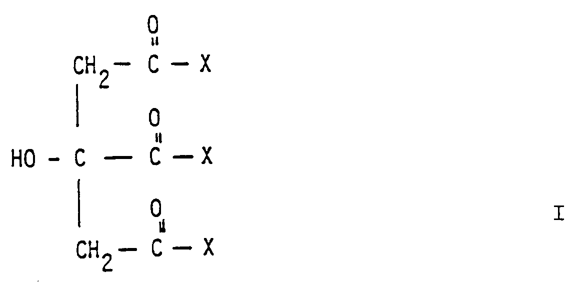
in which at least one X is (O-C₃H₆)₃-OCH₃ and any other X is OH.

In accordance with a second aspect, the present invention provides also a four components resinous system for a sheet molding compound, comprising
(a) an unsaturated polyester comprising a polycondensation product of one or more dihydric alcohols and one or more ethylenically unsaturated polycarboxylic acids;
(b) one or more low-profile thermoplastic polymers which cause phase separation and internal voids during a curing reaction;
(c) one or more olefinically unsaturated monomers which copolymerize with the unsaturated polyester; and,
(d) one or more components that remain compatible when the polyester and monomer cure and comprising an ester of the general formula 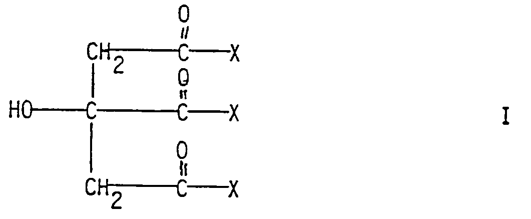 in which at least one X is (0-C₃H₆)₃-OCH₃ and any other X is OH.

The four component resinous system imparts improved surface smoothness when used with other known conventional ingredients for low-profile resin systems used in making sheet molding compositions.

The present invention relates to the discovery of the use in a group of components which remain compatible with a curing unsaturated polyester resin, and monomer used in a low-profile resin system. When these compatible components are included in combination with low-profile additives and used in sheet molding compositions, articles with very smooth surfaces may be molded. Additionally, the flow during the molding process is improved to the point that rapidly curing formulations may be composed, consequently the molding time is drastically reduced. Also, these compounds are helpful in controlling the thickening of SMC.

The unsaturated polyester component of the four component resinous system comprises the polycondensation reaction product of one or more dihydric alcohols and one or more ethylenically unsaturated polycarboxylic acids. By polycarboxylic acid is generally meant the polycarboxylic or dicarboxylic acids or anhydrides, polycarboxylic or dicarboxylic acid halides, and polycarboxylic or dicarboxylic esters. Suitable unsaturated polycarboxylic acids, and the corresponding anhydrides and acid halides that contain polymerizable carbon-to-carbon double bonds may include maleic anhydride, maleic acid, and fumaric acid. A minor proportion of the unsaturated acid, up to about forty mole percent, may be replaced by dicarboxylic or polycarboxylic acid that does not contain a polymerizable carbon-to-carbon bond. Examples of which include Ophthalic, isophthalic, terephthalic, succinic, adipic, sebacic and methyl-succinic. Dihydric alcohols that are useful in preparing the polyesters include 1,2-propane diol (hereinafter referred to as propylene glycol), dipropylene glycol, diethylene glycol, 1,3-butanediol, ethylene glycol and glycerol. Examples of suitable unsaturated polyesters are the polycondensation products of (1) propylene glycol and maleic and/or fumaric acids; (2) 1,3-butanediol and maleic and/or fumaric acids; (3) combinations of ethylene and propylene glycols (approximately 50 mole percent or less of ethylene glycol) and maleic and/or fumaric acid; (4) propylene glycol, maleic and/or fumaric acids and dicyclopentadiene reacted with water. In addition to the above described polyesters one may also use dicyclopentadiene modified unsaturated polyester resins as described in U.S. Patent No. 3,883,612. These examples are intended to be illustrative of suitable polyesters and are not intended to be all-inclusive. The acid number to which the polymerizable unsaturated polyesters are condensed is not particularly critical with respect to the ability of the low-profile resin to be cured to the desired product. Polyesters which have been condensed to acid numbers of less than 100 are generally useful, but acid numbers less than 70 are preferred. The molecular weight of the polymerizable unsaturated polyester may vary over a considerable range, but ordinarily those polyesters useful in the practice of the present invention have a molecular weight ranging from 300 to 5000, and more preferably, from 500 to 5000.

In preferred embodiments, the unsaturated polyester is present in amounts ranging from 20 to 45 percent, by weight, based on the total four component resinous system comprising the unsaturated polyester, the low-profile additive, monomer and compatible component containing one or more polyoxyethane substituents. Especially preferred concentrations of the unsaturated polyester are in the 28 to 35 percent, by weight, range.

Low-profile additives are materials that when mixed in an unsaturated polyester and cured, result in a multiphase system. If the low-profile additive and the unsaturated polyester are compatible (from the standpoint that a gross phase separation does not take place) before cure, the system is known as a one-pack. Those mixtures which tend to separate into two or more layers on standing are known as a two-pack resin systems. This does, however, necessitate mixing immediately before use. Some polymers that are useful as low-profile additives include homopolymers and copolymers of acrylic and methacrylic acid esters, cellulose acetate butyrate, vinyl acetate homopolymers and copolymers, polyurethanes prepared from polyisocyanates, preferably diisocyanates, and polyether polyols, numerous saturated polyesters, polycaprolactone, styrene-butadiene copolymers, some modified celluloses, and certain alkyl oxide polymers. The above list of low-profile additives is not intended to list all low-profile additives but rather to show examples of materials which have been used to cause the multiphase morphology present in low profile resins. In preferred embodiments the thermoplastic additive is present in amounts ranging from 5 to 30 percent, by weight, based on the total four component resinous system. Especially preferred concentrations of thermoplastic additive are in the 7 to 20 percent, by weight range.

The monomer component comprises materials that copolymerize with the unsaturated polyester. The olefinically unsaturated monomer that is copolymerizible with the unsaturated polyester is most generally styrene, however, methyl-styrene is also useful. In preferred embodiments the monomer is present in amounts ranging from 25 to 65 percent, by weight, based on the total four component resinous system. Especially preferred concentrations of monomer are in the 35 to 50 percent, by weight range.

In the present invention one or more components are added which are compatible with the unsaturated polyester and monomer during cure. According to the present invention, these compatible components give the added benefits of surface smoothness and better flowability, when compared with low-profile resin compositions without the compatible components. In the preferred embodiments the compatible component is present in amounts ranging from 0.5 to 15 percent, by weight, based on the total four component resinous sytem. Especially preferred concentrations of the compatible components are in the 1 to 8 percent, by weight range.

The compatible components of the present invention contains one or more polyoxyethane substituents having a general structure: 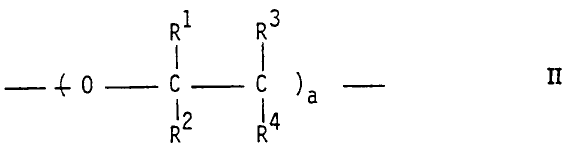
wherein R¹, R², R³, and R⁴, are selected from hydrogen, cycloalkyl, lower alkyl, phenyl, phenyl substituted by halogen, lower alkyl, acyl, or lower alkoxy; R¹, R², R³ and R⁴ may be the same or different; and a is an integer between 1 and 200, and in some embodiments a is less than 100 and in certain embodiments a is between 3 and 70.

The following terms used herein: "lower alkyl", "lower alkoxy", "lower phenyl", "cycloalkyl" and "acyl" generally contain from 1 to 50 carbons, as is well understood by those skilled in the art.

One example of compatible components that contain polyoxyethane substituents are polymers such as a polyalkylene oxide which has a molecular weight of between 200-5000. The molecular weight of the polyalkylene oxide polymer is such that the compatible component remains compatible with the curing unsaturated polyester and monomer. When the molecular weight of the polymer is too high, the polyalkylene oxide polymer is incompatible with the curing unsaturated polyester and monomer. At that point the polyalkylene oxide polymer acts like a low-profile additive component, which, by definition, is incompatible with the curing unsaturated polyester and monomer. Specific examples of polyalkylene oxide polymers useful as compatible components include polypropylene oxide having a molecular weight between 200-1000 and polyethylene oxide having a molecular weight between 200-5000.

Other examples of these compatible components include esters of citric acid, adipic acid and/or sebacic acid with tripropylene glycol monomethyl ether, dipropylene glycol monomethyl ether, diethylene glycol monomethyl ether and diethylene glycol monoethyl ether.

Examples of esters include triesters of a general structure: 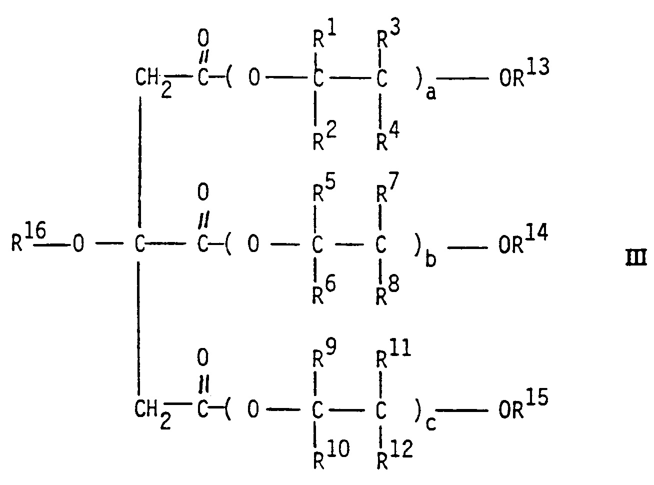
wherein R¹, R², R³, R⁴, R⁵, R⁶, R⁷, R⁸, R⁹, R¹⁰, R¹¹, R¹², R¹³, R¹⁴, R¹⁵ and R¹⁶ are selected from hydrogen, cycloalkyl, lower alkyl, phenyl, phenyl substituted by halogen, lower alkyl, acyl, or lower alkoxy, and R¹, R², R³, R⁴, R⁵, R⁶, R⁷, R⁸, R⁹, R¹⁰, R¹¹, R¹², R¹³, R¹⁴, R¹⁵, and R¹⁶ may be the same or different, a, b, and c are integers between 1 and 200, and a, b and c may be the same or different.

Specific examples of such triesters include wherein a = b = c = 3, R¹ = R² = R³ = R⁴ = R⁵ = R⁶ = R⁷ = R⁸ = R⁹ = R¹⁰ = R¹¹ = R¹² = H, R¹³ = R¹⁴ = R¹⁵ = CH₃, and R¹⁶ = H; and wherein a = b = c = 3, R¹ or R² or R³ or R⁴ = CH₃ and the others = H, R⁵ or R⁶ or R⁷ or R⁸ = CH₃ and the others = H, R⁹ or R¹⁰ or R¹¹ or R¹² = CH₃ and the others = H, R¹³ = R¹⁴ = R¹⁵ = CH₃ and R¹⁶ = H.

Still more examples of esters include diesters of a general structure: 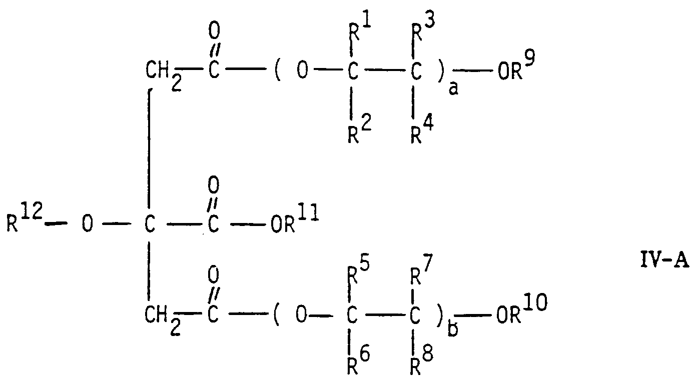
wherein R¹, R², R³, R⁴, R⁵, R⁶, R⁷, R⁸, R⁹, R¹⁰, R¹¹ and R¹² are selected from hydrogen, cycloalkyl, lower alkyl, phenyl, phenyl substituted by halogen, lower alkyl, acyl, or lower alkoxy, and R¹, R², R³, R⁴, R⁵, R⁶, R⁷, R⁸, R⁹, R¹⁰, R¹¹ and R¹² may be the same or different, a and b are integers between 1 and 200 and b may be the same or different.

Specific examples of such diesters include wherein a = b = 3, R¹ = R² = R³ = R⁴ = R⁵ = R⁶ = R⁷ = R⁸ = H, R⁹ = R¹⁰ = CH₃ and R¹¹ = R¹² = H; and wherein a = b = 3, R¹ or R² or R³ or R⁴ = CH₃ and the others = H, R⁵ or R⁶ or R⁷ or R⁸ = CH₃ and the others = H, R⁹ = R¹⁰ = CH₃ and R¹¹ = R¹² = H.

Still more specific examples of esters include diesters of a general structure: 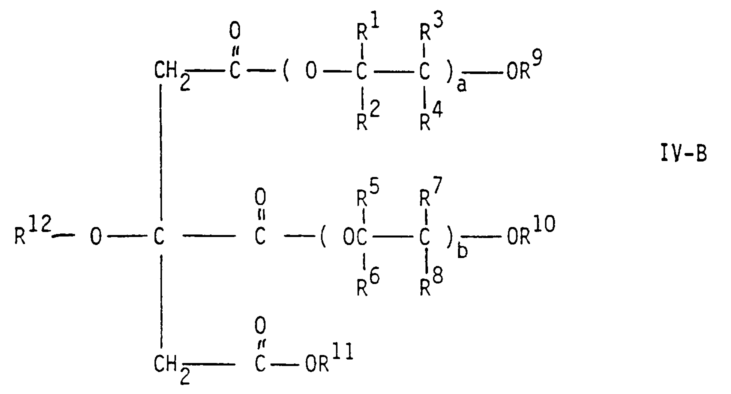
wherein R¹, R², R³, R⁴, R⁵, R⁶, R⁷, R⁸, R⁹, R¹⁰, R¹¹ and R¹² are selected from hydrogen, cycloalkyl, lower alkyl, phenyl, phenyl substituted by halogen, lower alkyl, acyl, or lower alkoxy, and R¹, R², R³, R⁴, R⁵, R⁶, R⁷, R⁸, R⁹, R¹⁰, R¹¹ and R¹² may be the same or different, a and b are integers between 1 and 200 and b may be the same or different.

Specific examples of such diesters include wherein a = b = 3, R¹ = R² = R³ = R⁴ = R⁵ = R⁶ = R⁷ = R⁸ = H, R⁹ = R¹⁰ = CH₃ and R¹¹ = R¹² = H; and wherein a = b = 3, R¹ or R² or R³ or R⁴ = CH₃ and the others = H, R⁵ or R⁶ or R⁷ or R⁸ = CH₃ and the others = H, R⁹ = R¹⁰ = CH₃ and R¹¹ = R¹² = H.

Still more specific examples of esters include monoesters of a general structure: 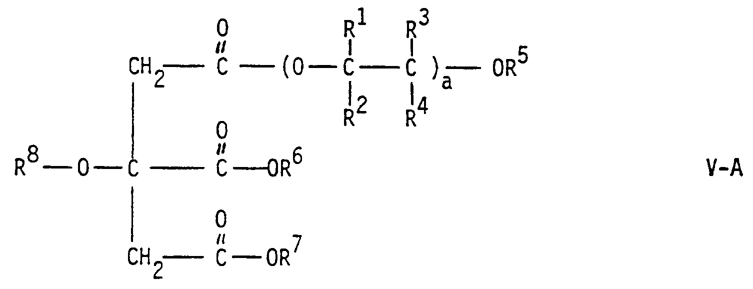
R¹, R², R³, R⁴, R⁵, R⁶, R⁷ and R⁸ are selected from hydrogen, cycloalkyl, lower alkyl, phenyl, phenyl substituted by halogen, lower alkyl, acyl, or lower alkoxy, and R¹, R², R³, R⁴, R⁵, R⁶, R⁷ and R⁸ may be the same or different, and a is an integer between 1 and 200.

Specific examples of such monoesters include wherein a = 3, R¹ = R² = R³ = R⁴ = H, R⁵ = CH₃ and R⁶ = R⁷ = R⁸ = H; and wherein a = 3, R¹ or R² or R³ or R⁴ = CH₃ and the others = H, R⁵ = CH₃ and R⁶ = R⁷ = R⁸ = H.

Still more specific examples of esters include monoesters of a general structure: 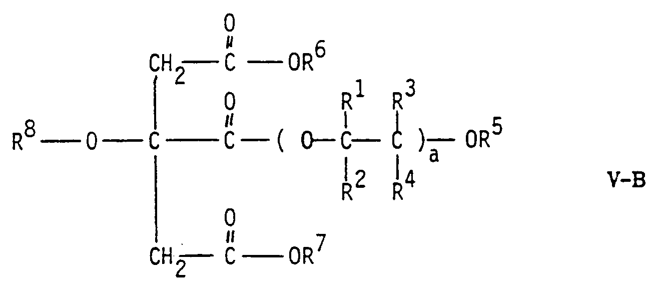
R¹, R², R³, R⁴, R⁵, R⁶, R⁷ and R⁸ are selected from hydrogen, cycloalkyl, lower alkyl, phenyl, phenyl substituted by halogen, lower alkyl, acyl, or lower alkoxy, and R¹, R², R³, R⁴, R⁵, R⁶, R⁷ and R⁸ may be the same or different, and a is an integer between 1 and 200.

Specific examples of such monoesters include wherein a = 3, R¹ = R² = R³ = R⁴ = H, R⁵ = CH₃ and R⁶ = R⁷ = R⁸ = H; and wherein a = 3, R¹ or R² or R³ or R⁴ = CH₃ and the others = H, R⁵ = CH₃ and R⁶ = R⁷= R⁸= H.

The invention also relates to a process for making citrate ester compositions of the general formula 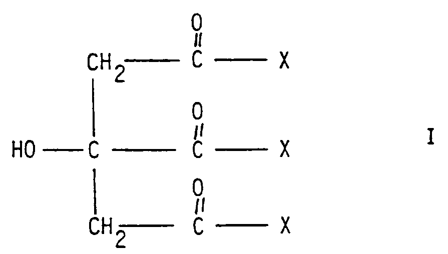
wherein X is selected from (O-C₃H₆)₃ OCH₃ and OH. The process comprises adding together 25-30 percent, by weight, of citric acid and 70 - 75 percent, by weight, of tripropylene glycol monomethyl ether, heating to a temperature in the range of 190°-240° C with a nitrogen sparge to remove water. The resulting product comprises 5 - 50 and preferably 32 - 42 percent, by weight, of 2-hydroxy-1,2,3-propane tricarboxylic acid-tripropylene glycol monomethylether triester, 5 - 95 and preferably 46 - 57 percent, by weight, of 2-hydroxy-1,2,3-propane tricarboxylic acid-tripropylene glycol monomethylether diester, and 5 - 50 and preferably 5 - 15 percent, by weight, of 2-hydroxy-1,2,3-propane tricarboxylic acid-tripropylene glycol monomethylether monoester.

The citrate esters described in this invention may also be useful as plasticizers for thermoplastic polymers such as polyvinyl chloride, or polystyrene.

The four component resinous system is suitable for mixing with other ingredients in order to form a sheet molding composition. For example, the four component resinous system is suitable for mixing with chemical thickeners which are physically mixed into the resin emulsion. The chemical thickeners generally include metal oxides, hydroxides and alkoxides of Group II, III or IV from the Periodic Table. Calcium oxide and magnesium oxide or the respective hydroxides are most often employed with four component resin compositions of the present invention. In preferred embodiments, the thickener is present in amounts ranging from 0.5 to 6 parts, by weight, based on the four component resinous system. The thickener is generally suspended in a carrier resin, as is known in the art. In preferred embodiments the carrier material comprises a composition which does not react with the thickener such as, for example, polymethylmethacrylate, polyvinylacetate, saturated or unsaturated polyesters, and similar materials well-known in the art. In preferred embodiments the carrier resin is present in amounts ranging from 0.5 to 8 parts, by weight, based on one hundred parts of the four component resinous system.

Catalysts are incorporated in small amounts into thermosetting polyester resins containing ethylenically unsaturated monomer to aid in curing or cross-linking the unsaturated polyester with the monomer. Such catalysts are well known and may be similarly utilized in this invention to aid in curing the unsaturated polyester and monomer mixed with the low-profile thermoplastic polymer. Typical catalysts, for example, include organic peroxides and peracids such as tertiary butyl perbenzoate, tertiary butyl peroctoate and benzoyl peroxide. The amounts of catalysts may be varied with the molding process and similarly varied with the level and types of inhibitors utilized, in a manner well known in the art. In preferred embodiments the catalyst is present in amounts ranging from 0.5 to 2.5 parts, by weight, based on one hundred parts of the four component resinous system.

Curing of the composition is carried out under heat and pressure typically, in closed, preferably positive pressure type molds. Mold release agents may be added to the compositions to perform their normal function, as is well understood in the art.

Fibers, fillers and pigments normally added to resin compositions can be likewise used in formulating the sheet molding composition of this invention. Reinforcing fibers or fibrous reinforcement is taken to mean glass fibers in one form or another, such as glass fabrics, chopped glass strands, chopped or continuous strand glass fiber mat; however, the terms also include reinforcing agents which may also be used if desired, for example, asbestos, cotton, synthetic organic fibers and metals. Fillers, usually inert, and inorganic material useful with the compositions of the present invention include, for example, clay, talc, calcium carbonate, silica, calcium silicate, and the like. In preferred embodiments the fillers are present in amounts ranging from 165 to 250 parts, by weight, based on one hundred parts of the four component resinous system.

Examples of pigments include carbon black, iron oxide and titanium dioxide, as well as organic pigments. In preferred embodiments the pigments are present in amounts ranging from 0 to 4 parts, by weight, based on one hundred parts of the four component resinous system.

The preparation of the sheet molding composition is generally carried out by blending together a first portion comprising the unsaturated polyester, the low-profile additive, the monomer, the compatible component, and such additives as a catalyst, mold release agent and fillers. This is generally known in the industry as the A-side formulation. The second portion (generally known as the B-side formulation) comprises the thickening agent and a carrier resin therefor, and such additives as pigments and mold release agents. In another aspect of the invention an additional monomer is added to the B-side formulation in which the thickener is suspended. In preferred embodiments the additional monomer comprises vinyl toluene or styrene. In preferred embodiments, the additional monomer is present in amounts ranging from 1 to 8 parts, by weight, based on one hundred parts of the four component resinous system.

The sheet molding composition can be prepared by mixing the components in a suitable apparatus at temperatures which are conventional and known to those skilled in the art. Once the sheet molding composition is formulated, the composition can be molded into thermoset articles having a desired shape. The actual molding cycle will, of course, depend upon the exact composition being molded. In preferred embodiments suitable molding cycles are conducted at temperatures ranging from 120°-177°C for periods of time ranging from 1/3 to 5 minutes.

The following formulations are provided to illustrate examples of the compositions of the present invention. All parts are parts by weight, unless otherwise expressly specified.

**TABLE I**

| Resin Compositions | | |
|---|---|---|
| Ingredients | Range (wt.%) | Preferred Range (wt.%) |
| Unsaturated polyester | 20-45 | 28-35 |
| Thermoplastic additive (low-profile) | 5-30 | 7-20 |
| Monomer | 25-65 | 35-50 |
| Compatible component | 0.5-15 | 1-8 |
| | 100 | 100 |

**TABLE II**

| Typical Sheet Molding Composition Formulation | | | | |
|---|---|---|---|---|
| Ingredients | Formulations | | | |
| | A | B | C | D |
| Resin | 100 | 100 | 100 | 100 |
| Catalyst | 1.5 | 1.5 | 1.5 | 1.5 |
| Release agent | 5.0 | 4.5 | 5.0 | 4.5 |
| Filler | 230 | 220 | 225 | 225 |
| Thickener | 4.0 | 5.0 | 4.5 | 4.8 |
| Pigment | 0.1 | 0.2 | 0.1 | 0.1 |
| Carrier | 1.55 | - | 1.5 | 1.6 |
| Secondary monomer | 5.6 | - | 5.5 | 5.5 |

The sheet molding compositions of the above formulations have shown unexpected improvements in surface aesthetics and mold fillout. These improvements are especially significant for use in sheet molding compounds (SMC). Moreover, increasingly thinner automobile parts are able to be molded with smoother surfaces than by any known systems.

For formulation A the unsaturated polyester comprises maleic anhydride and propylene glycol; the low-profile additive comprises a saturated polyester made from ethylene glycol and propylene glycol and adipic acid; the monomer comprises styrene; the compatible component comprises a polypropylene oxide having a molecule weight between 200 and 2000; the catalyst comprises tertiary butyl perbenzoate; added to the A-side, the release agent comprises calcium stearate and zinc stearate; the filler comprises calcium carbonate; the thickener comprises magnesium hydroxide; the carrier comprises polymethylmethacrylate; the pigment comprises a carbon black pigment suspension; and the secondary monomer comprises vinyl toluene.

Compression molded panels were made with each formulation with 27 percent, by weight, of 2.54 cm chopped glass fibers. When measured on a surface smoothness index instrument (LORIA® registered trademark of the Ashland Chemical Co.) the panels gave the LORIA® number of 60-70 as compared to the same formulation but without any compatible component which gave a number of 80-90. On the LORIA® instrument, the lower the number, the smoother the surface.

For formulation B the unsaturated polyester comprises maleic anhydride and propylene glycol; the low-profile additive comprises a saturated polyester made from ethylene glycol and propylene glycol and adipic acid; the monomer comprises styrene; the compatible component comprises a triester of citric acid with tripropylene glycol monomethyl ether; the catalyst comprises tertiary butyl perbenzoate; the release agent comprises calcium stearate; the filler comprises calcium carbonate; the thickener comprises magnesium hydroxide; and the pigment comprises a carbon black pigment suspension.

Compression molded panels made with Formulation B with 27 percent, by weight, of 2.54 cm chopped glass fibers. When measured on a surface smoothness index instrument (LORIA®) the panels gave a number of 55-60 as compared to the same formulation but without the compatible component which gave a number 80-90.

For formulation C the unsaturated polyester comprises maleic anhydride and propylene glycol; the low-profile additive comprises a saturated polyester made from ethylene glycol and propylene glycol and adipic acid; the monomer comprises styrene; the compatible component comprises polypropylene oxide having a molecular weight of 700 and citrate esters of the general formulae III, IV-A and IV-B, and V-A and V-B, wherein the polypropylene oxide comprises 3 percent and the citrate esters comprise 4 percent, by weight, of the resin formulation; the catalyst comprises tertiary butyl perbenzoate; the release agent comprises zinc stearate; the filler comprises calcium carbonate; the thickener comprises magnesium hydroxide; the carrier comprises polymethylmethacrylate; the pigment comprises a carbon black pigment suspension; and the secondary monomer comprises vinyl toluene.

Compression molded panels were made with formulation C with 27 percent, by weight, of 2.54 cm chopped glass fibers. When measured on a surface smoothness index instrument, LORIA ^{R} , the panels gave a number of 50 as compared to the same formulation without the compatible component which gave a number of 80-90.

For formulation D the unsaturated polyester comprises maleic anhydride and propylene glycol; the low profile additive comprises a saturated polyester made from ethylene glycol and propyleneglycol and adipic acid; the monomer comprises styrene; the compatible component comprises polypropylene oxide having a molecular weight of 700 and citrate esters of the general formulae III, IV-A, IV-B and V-A and V-B wherein the polypropylene oxide comprises 3 percent and the citrate esters comprise 4 percent, by weight, of the resin formulation; the catalyst comprises tertiary butyl perbenzoate; the release agent comprises calcium stearate; the filler comprises calcium carbonate; the thickener comprises 4 parts per hundred resin magnesium hydroxide and 0.8 parts per hundred magnesium oxide; the carrier comprises polyvinylacetate; the pigment comprises a carbon black pigment suspension; and the secondary monomer comprises vinyl toluene.

Compression molded panels were made with formulation D with 27 percent, by weight, of 2.54 cm chopped glass fibers. When measured on a surface smoothness index instrument, LORIA ^{R}, the panels gave a number of 48 as compared to the same formulation without the compatible components which gave a number of 80-90.

## Claims

1. A compound of the formula 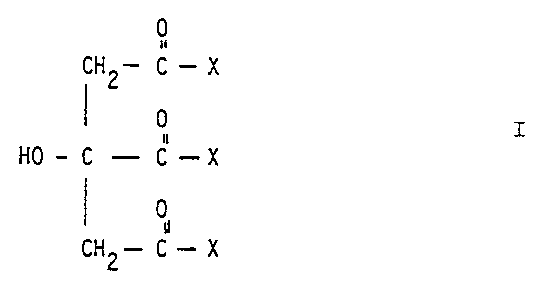 in which at least one X is (O-C₃H₆)₃-OCH₃ and any other X is OH.

2. A compound according to claim 1, having the formula 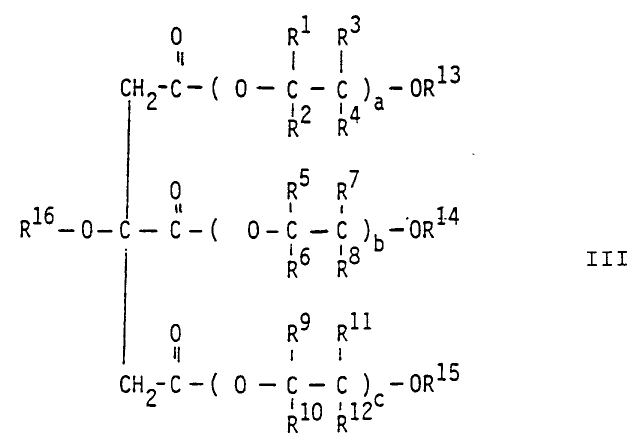 in which each of a, b and c is 3, one of R¹, R², R³ and R⁴ is CH₃ and the others are H, one of R⁵, R⁶, R⁷ and R⁸ is CH₃ and the others are H, one of R⁹, R¹⁰, R¹¹ and R¹² is CH₃ and the others are H, each of R¹³, R¹⁴ and R¹⁵ is CH₃ and R¹⁶ is H.

3. A four component resinous system for a sheet molding composition comprising:
(a) an unsaturated polyester comprising a polycondensation product of one or more dihydric alcohols and one or more ethylenically unsaturated polycarboxylic acids;
(b) one or more low-profile thermoplastic polymers which cause phase separation and internal voids during a curing reaction;
(c) one or more olefinically unsaturated monomers which copolymerises with the unsaturated polyester; and
(d) one or more compatible components comprising an ester according to claim 1.

4. A resinous system according to claim 3, wherein the compatible components comprise a mixture of 2-hydroxy-1,2,3-propane tricarboxylic acid-tripropylene glycol monomethylether triester, diesters and monoesters.

5. A resinous system according to claim 3, wherein the compatible component comprises polypropylene oxide having a molecular weight of 700 and a mixture of 2-hydroxy-1,2,3-propane tricarboxylic acid-tripropylene glycol monomethylether triester, diesters and monoesters.

6. A resinous system according to any one of claims 3 to 5, wherein the unsaturated polyester comprises the polycondensation product of dihydric alcohols and an ethylenically unsaturated polycarboxylic acid.

7. A resinous system according to any one of claims 3 to 5, wherein the unsaturated polyester comprises the polycondensation product of maleic and/or fumaric acids and propylene glycol; the polycondensation product of 1,3-butanediol and maleic and/or fumaric acids; the polycondensation product of ethylene and propylene glycols comprising approximately 50 mole percent or less of ethylene glycol, and maleic and/or fumaric acids; the polycondensation product of propylene glycol, maleic and/or fumaric acids and dicyclopentadiene reacted with water; or, the polycondensation product of propylene glycol, maleic and/or fumaric acids and isophthalic acid.

8. A resinous system according to any one of claims 3 to 7, wherein the low-profile thermoplastic polymer comprises a reaction product of ethylene glycol and propylene glycol and adipic acid; a polyvinyl acetate homopolymer or copolymer; or, a polymethylmethacrylate.

9. A resinous system according to any one of claims 3 to 8, wherein the monomer comprises styrene; methyl-styrene; or vinyl toluene.

10. A resinous system according to any one of claims 3 to 9, wherein the unsaturated polyester is present in an amount of 20-45 percent by weight of the four component resin; the low-profile thermoplastic polymer is present in an amount of 5-30 percent by weight of the four component resin; the monomer is present in an amount of 25-65 percent by weight of the four component resin; and the compatible component is present in an amount of 0.5-15 percent by weight of the four component resin.

11. A resinous system according to claim 10, wherein the unsaturated polyester is present in an amount of 28-35 percent by weight of the four component resin; the low-profile thermoplastic polymer is present in an amount of 7-20percent by weight of the four component resin; wherein the monomer is present in an amount of 35-65 percent by weight of the four component resin; and the compatible component is present in an amount of 1-8 percent by weight of the four component resin.

12. A process for making a citrate ester according to claim 1, which comprises reacting together 25-30 percent by weight of citric acid and 70-75 percent by weight of tripropylene glycol monomethyl ether at a temperature of 190°-240°C.

## Patentansprüche

1. Verbindung der Formel 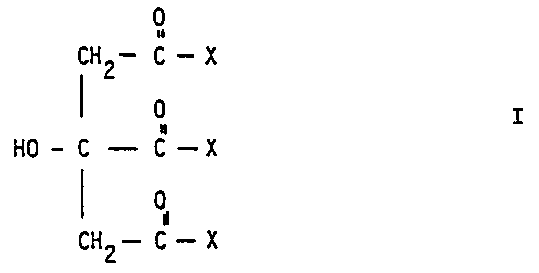 in der mindestens eines der Symbole X (-O-C₃H₆)₃-OCH₃ ist und weitere Symbole X -OH bedeuten.

2. Verbindung nach Anspruch 1 der Formel 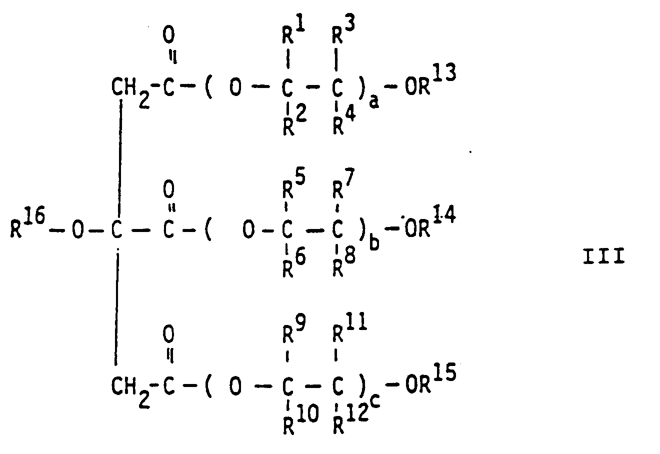 in der jedes von a, b und c 3 bedeutet, eines der Symbole R¹, R², R³ und R⁴ -CH₃ ist und die anderen H bedeuten, eines der Symbole R⁵, R⁶, R⁷ und R⁸ -CH₃ ist und die anderen H bedeuten, eines der Symbole R⁹, R¹⁰, R¹¹ und R¹² -CH₃ ist und die anderen H bedeuten, jedes von R¹³, R¹⁴ und R¹⁵ -CH₃ ist und R¹⁶ für H steht.

3. Vierkomponenten-Harzsystem für eine Plattenformmasse, enthaltend
(a) einen ungesättigten Polyester, der ein Polykondensationsprodukt aus einem oder mehr zweiwertigen Alkoholen und einem oder mehr ethylenisch ungesättigten Polycarbonsäuren darstellt,
(b) ein oder mehr schrumpfarme, thermoplastische Polymere, die während der Härtungsreaktion Phasentrennung und die Bildung von inneren Hohlräumen verursachen,
(c) ein oder mehr olefinisch ungesättigte Monomere, die mit dem ungesättigten Polyester copolymerisieren, und
(d) ein oder mehr damit verträgliche Komponenten, die einen Ester nach Anspruch 1 umfassen.

4. Harzsystem nach Anspruch 3, wobei die verträglichen Komponenten ein Gemisch aus 2-Hydroxy-1,2,3-propan-tricarbonsäure-tripropylenglykol-monomethylether-triester, den entsprechenden Diestern und Monoestern umfassen.

5. Harzsystem nach Anspruch 3, wobei die verträgliche Komponente Polypropylenoxid mit einem Molekulargewicht von 700 und ein Gemisch aus 2-Hydroxy-1,2,3-propan-tricarbonsäure-tripropylenglykol-monomethylether-triester, den entsprechenden Diestern und Monoestern umfaßt.

6. Harzsystem nach einem der Ansprüche 3 bis 5, wobei der ungesättigte Polyester das Polykondensationsprodukt von zweiwertigen Alkoholen mit einer ethylenisch ungesättigten Polycarbonsäure umfaßt.

7. Harzsystem nach einem der Ansprüche 3 bis 5, wobei der ungesättigte Polyester das Polykondensationsprodukt von Maleinsäure und/oder Fumarsäure und Polypropylenglykol, das Polykondensationsprodukt von 1,3-Butandiol und Maleinsäure und/oder Fumarsäure, das Polykondensationsprodukt von Ethylenglykol und Propylenglykol mit etwa 50 Mol-% oder weniger an Ethylenglykol und Maleinsäure und/oder Fumarsäure, das Polykondensationsprodukt von Propylenglykol, Maleinsäure und/oder Fumarsäure und mit Wasser umgesetztem Dicyclopentadien oder das Polykondensationsprodukt von Propylenglykol, Maleinsäure und/oder Fumarsäure und Isophthalsäure umfaßt.

8. Harzsystem nach einem der Ansprüche 3 bis 7, wobei das schrumpfarme thermoplastische Polymere ein Reaktionsprodukt von Ethylenglykol und Propylenglykol mit Adipinsäure, ein Homopolymer oder Copolymer von Polyvinylacetat oder Polymethylmethacrylat umfaßt.

9. Harzsystem nach einem der Ansprüche 3 bis 8, wobei das Monomere Styrol, Methylstyrol oder Vinyltoluol umfaßt.

10. Harzsystem nach einem der Ansprüche 3 bis 9, wobei der ungesättigte Polyester in einer Menge von 20 bis 45 Gew.-% des Vierkomponentenharzes, das schrumpfarme thermoplastische Polymere in einer Menge von 5 bis 30 Gew.-% des Vierkomponentenharzes, das Monomere in einer Menge von 25 bis 65 Gew.-% des Vierkomponentenharzes und die verträgliche Komponente in einer Menge von 0,5 bis 15 Gew.-% des Vierkomponentenharzes vorhanden ist.

11. Harzsystem nach Anspruch 10, wobei der ungesättigte Polyester in einer Menge von 28 bis 35 Gew.-% des Vierkomponentenharzes, das schrumpfarme thermoplastische Polymere in einer Menge von 7 bis 20 Gew.-% des Vierkomponentenharzes, das Monomere in einer Menge von 35 bis 65 Gew.-% des Vierkomponentenharzes und die verträgliche Komponente in einer Menge von 1 bis 8 Gew.-% des Vierkomponentenharzes vorhanden ist.

12. Verfahren zur Herstellung eines Citratesters nach Anspruch 1, das darin besteht, daß 25-30 Gew.-% Zitronensäure und 70-75 Gew.-% Tripropylenglykolmonomethylether bei einer Temperatur von 190 - 240 °C umgesetzt werden.

## Revendications

1. Composé répondant à la formule : 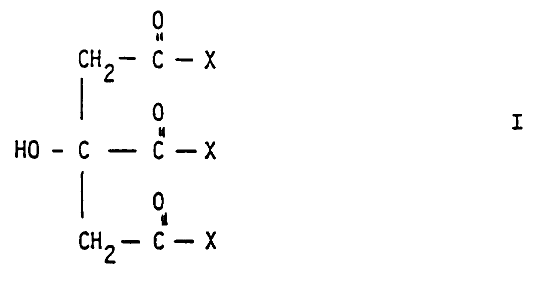 dans laquelle au moins un X est (O-C₃H₆)₃-OCH₃ et tout autre X est OH.

2. Composé selon la revendication 1, répondant à la formule : 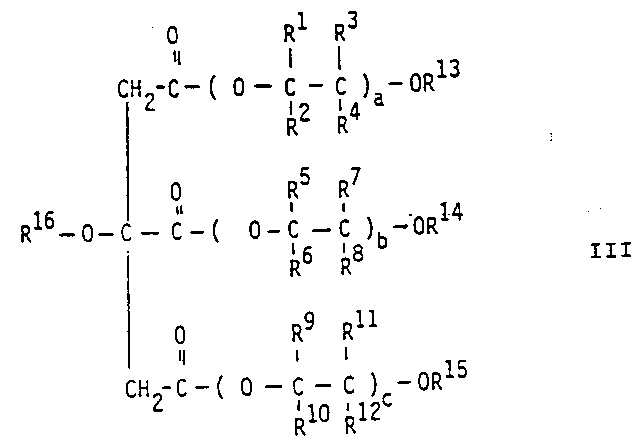 dans laquelle chacun des indices a, b et c est égal à 3, un des groupes R¹, R², R³ et R⁴ est CH₃ et les autres sont H, un des groupes R⁵, R⁶, R⁷ et R⁸ est CH₃ et les autres sont H, un des groupes R⁹, R¹⁰, R¹¹ et R¹² est CH₃ et les autres sont H, chacun des groupes R¹³, R¹⁴ et R¹⁵ est CH₃ et R¹⁶ est H.

3. Système résineux à quatre constituants, pour une composition de moulage de feuille, comprenant :
(a) un polyester insaturé comprenant un produit de polycondensation d'un ou plusieurs dialcools, et d'un ou plusieurs acides polycarboxyliques à insaturation éthylénique ;
(b) un ou plusieurs polymères thermoplastiques à stabilité dimensionnelle élevée, qui provoque une séparation de phases et des vides internes au cours de la réaction de durcissement ;
(c) un ou plusieurs monomères à insaturation oléfinique qui se copolymérisent avec le polyester insaturé ; et
(d) un ou plusieurs constituants compatibles comprenant un ester selon la revendication 1.

4. Système résineux selon la revendication 3, dans lequel les constituants compatibles comprennent un mélange de triester, de diesters et de monoesters de l'acide 2-hydroxy-1,2,3-propane tricarboxylique et de l'éther monométhylique du tripropylèneglycol.

5. Système résineux selon la revendication 3, dans lequel le constituant compatible comprend un poly(oxyde de propylène) ayant une masse moléculaire de 700 et un mélange de triester, de diesters et de monoesters de l'acide 2-hydroxy-1,2,3-propane tricarboxylique et de l'éther monométhylique du tripropylèneglycol.

6. Système résineux selon l'une quelconque des revendications 3 à 5, dans lequel le polyester insaturé comprend le produit de polycondensation de dialcools et d'un acide polycarboxylique à insaturation éthylénique.

7. Système résineux selon l'une quelconque des revendications 3 à 5, dans lequel le polyester insaturé comprend le produit de polycondensation des acides maléïque et/ou fumarique et du propylèneglycol ; le produit de polycondensation du 1,3-butanediol et des acides maléique et/ou fumarique ; le produit de polycondensation des éthylène- et propylène glycols comprenant environ 50 % en moles ou moins d'éthylèneglycol et des acides maléique et/ou fumarique ; le produit de polycondensation du propylèneglycol, des acides maléique et/ou fumarique et du dicyclopentadiène ayant réagi avec l'eau ; ou le produit de polycondensation du propylèneglycol, des acides maléique et/ou fumarique et de l'acide isophtalique.

8. Système résineux selon l'une quelconque des revendications 3 à 7, dans lequel le polymère thermoplastique à stabilité dimensionnelle élevée comprend un produit de la réaction de l'éthylèneglycol et du propylèneglycol et de l'acide adipique ; un homopolymère ou copolymère poly(acétate de vinyle) ; ou un poly(méthacrylate de méthyle).

9. Système résineux selon l'une quelconque des revendications 3 à 8, dans lequel le monomère comprend le styrène, le méthylstyrène, ou le vinyltoluène.

10. Système résineux selon l'une quelconque des revendications 3 à 9, dans lequel le polyester insaturé est présent dans une proportion de 20 à 45 % en poids de la résine à quatre constituants ; le polymère thermoplastique à stabilité dimensionnelle élevée est présent dans une proportion de 5 à 30 % en poids de la résine à quatre constituants ; le monomère est présent dans une proportion de 25 à 65 % en poids de la résine à quatre constituants ; et le constituant compatible est présent dans une proportion de 0,5 à 15 % en poids de la résine à quatre constituants.

11. Système résineux selon la revendication 10, dans lequel le polyester insaturé est présent dans une proportion de 28 à 35 % en poids de la résine à quatre constituants ; le polymère thermoplastique à stabilité dimensionnelle élevée est présent dans une proportion de 7 à 20 % en poids de la résine à quatre constituants, le monomère est présent dans une proportion de 35 à 65 % en poids de la résine à quatre constituants ; et le constituant compatible est présent dans une proportion de 1 à 8 % en poids de la résine à quatre constituants.

12. Procédé de préparation d'un ester citrique selon la revendication 1, qui comprend la réaction de 25 à 30 % en poids d'acide citrique et 70 à 75 % en poids d'éther monométhylique du tripropylèneglycol à une température de 190 à 240 °C.
